# EUROPEAN PATENT APPLICATION

(11) **EP 1 923 701 A1**
(43) Date of publication of application: **21.05.2008**
(21) Application number: 06077041.9
(22) Date of filing: 17.11.2006
(51) Int. Cl.: G01N 33/50

(54) **Method for ligand screening with microcapsules as nanoreactor**

(71) Applicant: Spinnovation Holding BV, 6525 ED Nijmegen (NL); Encapson B.V., 6525 EC Nijmegen (NL)
(72) Inventor: The designation of the inventor has not yet been filed
(74) Representative: van Loon, C.J.J.

(57) **Abstract**

The present invention is directed to a method for ligand screening, which method comprises a step wherein a potential ligand is brought into contact with a target compound, characterized in that said target is encapsulated by a semi-porous shell. The potential interaction of the ligand and the target can be established by a detection step, which is based on for instance fluorescence spectroscopy, NMR spectroscopy, surface plasmon resonance, LC/MS-based detection method, or combinations thereof.

## Description

The invention is directed to methods for ligand screening, in particular to methods for using target molecules for testing their interaction with potential ligands. The target molecules can be proteins, enzymes, nucleic acids, polymers, antibodies, antigens, parts of a cell, microsomes, viruses and the like. The ligands can be small organic molecules, natural compounds, peptides, new chemical entities, small proteins, small nucleic acids, organo-metallic compounds, small polymers and the like. In the method of the present invention the target molecules are immobilized in a novel way to improve screening efficiency.

One of the main challenges currently faced by pharmaceutical companies is the essential need to identify new lead compounds (potential source of new drugs). After identification, the structure of these molecules must be optimized to improve their ability to interact with their intended target and trigger an appropriate biological response. A major issue here is the delivery of a sufficient number of new molecules to the drug discovery pipeline, which is a necessary condition to insure increased chance to identify, and further develop, a good drug candidate.

There has been a steadily growing demand for new technologies and applications enabling for faster and more accurate screening dedicated to the identification of new lead compounds. To address this issue, many high-throughput screening approaches (HTS) have been developed. These methods are based on automated or semi-automated screening of large compound libraries (typically ranging from thousands to hundreds of thousand of compounds) in which their interaction with a defined target, often a protein or enzyme, is tested.

The overall principle of these known screening methods is to bring the potential ligand(s) in contact with the target and assess whether they interact (bind). This binding interaction can be directly assessed by analytical techniques based on, for instance, fluorescence spectroscopy, X-ray crystallography or even NMR spectroscopy. Alternatively, the binding interaction can be indirectly assessed via techniques such as surface plasmon resonance, or LC/MS-based analytical applications. For instance, fluorescence and radiation based HTS are nowadays a fully integrated part of every lead discovery program.

A serious limitation of these known screening methods is that the compounds usually need to display a medium- to high-binding affinity and/or a low IC50 (half maximal inhibitory concentration), typically IC50<10µM. For this reason, many HTS methods fail to identify compounds which may bind weakly but which would nonetheless display high selectivity for the target.

Another limitation is that this random approach is costly in terms of ligand and target consumption, and often requires many rounds of analysis to reach statistically relevant results, considering the limits of reproducibility of these assays.

To overcome this, alternative screening approaches have been suggested, for instance those based on computer aided design (virtual screening) to reduce the library size. Also detection methods such as NMR spectroscopy have been used, which is capable of detecting weak binding interactions. Although the NMR approach is characterized by a low turn over (low throughput) and low sensitivity, the libraries to be screened can be reduced to less than 3 000 compounds by combining it with virtual screening approaches. Moreover, NMR is not only able to detect weak binders (binding in the millimolar range), but is also able to provide much more detailed, accurate and reproducible results when addressing binding interaction issues.

Once a lead compound has been identified, based on its affinity and/or specificity for a defined target, it needs to be further optimized. Its structure and thus its physico-chemical properties are subsequently tested and modified to improve its specificity, bioavailability, stability, and to reduce its toxicity. In this optimization phase of a new lead compound there is again a need for screening methods that can provide an accurate feedback on the compound's improved performance or "drug-ability" against the target before it can reach the clinical phase.

Enhancement of the screening throughput and reduction of sample consumption in screening assays can be achieved by immobilizing the target. Known means for immobilizing target compounds comprise for instance direct chemical attachment (tethering) of the target compound to a substrate and matrix entrapment, as will be explained in more detail herein below.

Once the target has been immobilized, the ligand - or mixture of ligands - is brought to the vicinity of the target - typically by letting the potential ligand(s) diffuse towards the target - while the interaction between the ligand(s) and the target is being detected. Once the measurement/detection of the potential interaction or absence of interaction has been performed, the ligand can be further eluted and replaced by a new one to test its affinity to the target. In such a way, the same target can be tested over and over against many different ligands. This is usually done in an automated fashion, reducing the amount of target compound required for the assay and potentially allowing for recycling the eluted ligands. This principle is used in many screening applications using different detection techniques such as using Surface Plasmon Resonance (*e.g.* BIACORE™), MS (*e.g.* Frontal Affinity Chromatography coupled to mass spectrometry, Schriemer D.C., Angew. Chem. Int. Ed. 1998, 37, 3383-3387), fluorescence spectroscopy (*e.g.* Fluorescence Resonance Energy Transfer, Stenroos et al., Cytohin,e 1998, 10, 495-499; Fluorescence Polarization, Jameson et al., 2003 Comb. Chem. High Throughput Screen 2003, 6, 167-176) or even NMR spectroscopy ("Target Immobilized NMR Screening" or "TINS", Vanwetswinkel et al., Chem. Biol. 2005, 12, 207-216).

Known target immobilization strategies rely on chemical and/or biochemical modification of the target, such as coupling the target to biotin (biotinylation) or functionalization of the N- or C-terminal of a protein sequence. A chemically biotinylated target will interact with a surface, typically glass or resin beads (*e.g.* Sepharose) coated with either streptavidin or avidin. The tight biotin-streptavidin or biotin-avidin interaction ensures that the target remains strongly attached to the solid surface.

Another way to achieve immobilization is to express the target protein with a tag (*e.g.* a His-tag). It can then be immobilized by direct interaction with antibodies (such as anti-His), the antibodies themselves being attached to the bead surface by means of a streptavidin-biotin interaction.

A third alternative is to chemically link the target to the bead surface using functionalized linkers. For instance, in the case of proteins, beads coated with linkers presenting carboxylic acid groups can directly interact with the protein N-terminal tail. This immobilization method is typically irreversible, as opposed to the biotinylation or an antibody approach, which are based on a potentially reversible interaction.

Although these immobilization approaches are commonly used, they carry intrinsic limitations. First of all, for an accurate assay, the target's activity must remain unaffected by the immobilization method. In the case of direct chemical/biochemical modifications of the target, there may well be an alteration of the target's structure, which can adversely affect its activity and thus the overall validity of the assay. When non-covalent biochemical interactions are used, such as biotin-streptavadin or antibody interaction with the target, there could also be an alteration of the target structure and therefore its activity. It is difficult to predict or determine beforehand whether immobilizing the target via known approaches is likely to affect, or cause complete loss of its activity.

Another drawback is that, considering such immobilization approaches, certain targets would be difficult or simply impossible to immobilize. Targets such as membrane bound receptors (GPCR, Toll-like receptors, etc.) or complex systems such as microsomes are known to be difficult or impossible to immobilize in this way. Many targets are also potentially unstable and would degrade over a short period of time. In such cases, fresh binding assays would have to be prepared and rapidly used within a limited time frame, limiting the types of chemistry/biochemistry which can be used. These targets can also be prone to degradation either spontaneously, caused by external degrading agents (proteases, DNases, RNases, fungi), or resulting from irreversible interactions with some ligands used during the screening. Also the buffer conditions (temperature, pH) must be compatible with both the immobilization method and the target stability.

An alternative to direct attachment of the target to a surface is to immobilize a target by entrapment in a matrix. Silicate-based sol-gel matrices have been shown to be suitable for entrapment of biological targets. These porous matrices are prepared by polymerization of an inorganic silicate mixture in which the targets are already suspended. The reaction conditions used are relatively mild, so that most sturdy enzymes or enzyme complexes, immobilized in such a way retain their native activity. However, there are also reports of a decrease or inhibition of activity in the case of less robust enzymes, showing that this method has its limitations. For instance, in some cases, the production of ethanol or methanol during the polymerization process of the gel can be a serious limiting factor, affecting the target and leading to changes in binding, catalytic and/or inhibition constants (Besanger et al., Anal. Chem. 2003, 75, 2382-2391). This type of immobilization can, however, quickly be tested on any new system with limited costs, since no expensive chemicals are required. The porous gel allows entrapped targets to be reached by small ligands which after being loaded on the surface of the gel can diffuse through to the target (Veith et al., J. Controlled Release 2004, 99, 315-327). It has also been shown that some entrapped systems confer an increased shelf-life time to the assay (Sakai-Kato et al., Anal. Chem. 2005, 77, 7080-7083).

There are, however, several limitations to the use of silicate-based sol-gel matrices as immobilization technique. Although the formation of the gel around the targets uses mild chemistry it is still not suitable for use with extremely sensitive targets. Also diffusion of the ligand through the gel must be fast, while diffusion of the target must be absent or extremely slow. This balance is in some cases extremely hard to reach. Another limitation is the fact that the solvents themselves which are run over the gel can affect the integrity of the gel, thus reducing the retention properties and allowing the target to diffuse out.

Despite high expectations, HTS as a general approach has failed to deliver most of its prospects: the number of active new chemical entities (NCE) found from large combinatorial libraries, is far lower than what was initially expected. The use of more rational approaches like virtual screening has helped to improve this, but there are still some important limitations. Material and miniaturization costs are quite high, large quantities of targets are often required, target stability is limited - even when "protected" in a gel - and target immobilization can have an unknown effect on the outcome of the assay.

It is an objective of the present invention to address the issues highlighted above, in particular the issues related to target immobilization. The present invention seeks to provide an alternative to current target immobilization methods, in particular applied to ligand screening assays, which solves, at least in part, the above-mentioned drawbacks of the prior art immobilization methods.

It was found that when a target compound is encapsulated within a semi-permeable shell, this objective can be met. Thus, in a first aspect, the present invention is directed to a method for ligand screening, comprising the step of contacting a potential ligand with a target compound, wherein said target compound is encapsulated within a semi-permeable shell.

Semi-permeable in this respect means that the shell is partially permeable, *viz.* the shell allows the passage of certain molecules, but acts as a barrier to others. Whether a certain molecule passes the shell or not is determined, for instance, on molecular weight.

The porous shell generally comprises amphiphilic molecules, in particular lipids, or polymeric materials, in particular homopolymers or block copolymers.

Shells comprising homopolymers and block copolymers can be conveniently made using known techniques, for example emulsion polymerization, spray drying, electro deposition, ion sputtering, crystallization, or preparation of a spherical particle from which the core is removed.

More preferred are shells comprising block copolymers in which the molecules comprise a hydrophobic part and a hydrophilic part. These polymers can be used to prepare polymeric capsules, such as vesicular structures (*viz.* capsules comprising a bilayer) and micellar structures (*viz*. ball-like structures wherein the hydrophobic "tails" of the block copolymer of the molecules cluster into the center and the hydrophilic "heads" of the block copolymer are situated at the outer surface and thus form the corona). The type of capsule that is formed can be controlled using known techniques (Walde et al., Biomol. Eng. 2001, 18, 143-177).

The pore size of the capsules is an important parameter according to the present invention. Generally speaking, the pore size should be small enough for the target compounds to remain entrapped in the capsules. On the other hand, the pore size must be large enough to allow for diffusion of ligand compounds through the pores. The pore size should be such that the desired cut-off size for the target compound is obtained. A suitable cut-off size is 1 500 D, *viz.* targets larger than 1 500 D are kept inside the shell, whereas smaller target compounds can move in and out of the shell, *e.g.* through diffusion. To this end, the average pore diameter is typically in the order of several nm, *e.g.* from 0.1 to 5 nm, preferably from 0.3 to 1 nm. The pore size can be adjusted using known means, in particular by crosslinking the shell (Vriezema et al., Angew. Chem. Int. Ed. 2003, 42, 772-776).

Semi-permeable shells that can be used in accordance with the present invention and their preparation are for instance disclosed in WO-A-2006/049497, incorporated herein by reference. According to this document, capsules are made by allowing a vesicle to be formed from an amphiphilic block copolymer, comprising both hydrophilic and hydrophobic groups as discussed hereinabove, which copolymer comprises cross-linkable groups. The vesicles are stabilized by cross-linking the cross-linkable groups. WO-A-2006/049497 describes thiophene cross-linkable groups, but other groups are possible, such as acids, alcohols, amines, alkynes, alkenes, and azides.

The size of the porous shell particles is not particularly critical, and can range for instance from 50 nm to 150 µm, *e.g.* about 0.5 µm.
Figure 1 schematically depicts capsules trapped in a sol gel, in accordance with the present invention.
Figure 2 schematically depicts capsules tethered to a surface, in accordance with the present invention.
Figure 3 shows a transmission electron micrograph of ubiquitin filled PS-PIAT (polystyrene-*b*-polyisocyanoalanyl thiophene) polymer capsules, prepared in accordance with the present invention.
Figure 4 shows a comparison of ¹H NMR spectra of A) free ubiquitin protein in solution and B) encapsulated ubiquitin protein.
Figure 5 shows a comparison of diffusion properties of A) free ubiquitin protein in solution and B) ubiquitin protein encapsulated, measured via pulsed-field gradient NMR spectroscopy. The encapsulated molecules (B) display a much stronger NMR signal than the free ones (A) after strong gradients have been applied. It shows that the encapsulated proteins diffuse much slower than the non-encapsulated ones. Typically, the encapsulated proteins move at the speed of the capsules free in solution.

In accordance with the present invention the target compound may be captured by the porous shell, for instance while being present in solution during shell formation *via* self assembly or spray-drying. The shell can be made permeable *via* swelling or introduction of trans-membrane proteins, which then allows the compound to diffuse inside the shell, the pores can subsequently be closed. Alternatively, micro injection can be used to directly inject the target compound into the shell. All these methods are further detailed by Vriezema et al. in Chem. Rev. 2005, 105, 1455-1490 and all the references therein.

According to the present invention the target advantageously remains in its native (solvated) state due to the encapsulation, meaning that its structure remains in its native form, and can be directly investigated using spectroscopic methods, such as for e.g. fluorescence or NMR spectroscopy. Also the lifetime of the target is enhanced as its exposure to external degrading agents is reduced. The materials forming the membrane of the capsule can be chosen such as to not interfere with the signal (*e.g.* the NMR signal) of the target or the target/ligand complex itself.

In one embodiment of the present invention, the free capsules containing the target are directly used in suspension *e.g.* in an aqueous solvent. Small compounds can be added to the suspension, which can then diffuse through the pores of the free capsule and reach the target, while the target stays trapped inside, based on a pore size exclusion principle.

In another embodiment, encapsulated targets are subsequently immobilized in matrices such as in a sol-gel, which is then subjected to screening, by letting the ligand or the mixture of ligands diffuse through the gel and capsule pores to reach the target. The encapsulation step in this embodiment of the present invention uses milder conditions than (direct) sol-gel preparation. As a result, more sensitive targets can be screened in accordance with the present invention than when the targets are brought into the sol-gel directly. Also, because the polymer capsules are much larger than the targets, diffusion of the encapsulated targets is much less an issue than would be the case with non-encapsulated targets. The rate of cross-linking within the gel can thus be much lower and still be efficient. Also the shelf-life of the encapsulated targets is higher than of targets directly immobilized in sol-gels.

In yet another embodiment the capsules, containing the targets, are subsequently physically immobilized on a surface. This can be done in several ways, using either a reversible non-covalent interaction, such as a biotin/streptavidin system, or a covalent interaction in which the capsules are chemically bound to the surface.

This can be done by functionalizing the surface of the polymer shell via the addition of a percentage of functionalized block copolymer, for instance between 0.1 and 70 wt.% functionalized, more preferably between 0.5 and 30 wt.%, even more preferably between 1 and 15 wt %.

This can also be done by the addition of a functional homopolymer during the formation of the shell, allowing the homopolymer to be incorporated and to give a functional handle to the surface of the shell. This should also be between 0.1 and 70 wt.% functionalized, more preferably between 0.5 and 30 wt.%, even more preferably between 1 and 15 wt%. Any functionality which can bind either non-covalently or covalently with another functionality on a surface can be used.

Non-covalent functionalities consist of any functionality which can interact either through hydrogen bonding or complexation with a second functionality on a surface. Examples of such interactions are biotin and streptavidin, nucleo-bases, cyclodextrindextrin and adamantane, hydrogen bonded 2-ureido-4[1H]-pyrimidinone dimers, metal complexes, protein-protein interactions, Π-Π interactions, Van der Waals interactions, more preferably streptavidin and biotin.

Functionalities which can be used to covalently bind the polymer shells to the surface can be chosen from the group consisting of acids, azides, amines, acid chlorides, acid bromides, chlorides, bromides, alkynes, alkenes, alcohols, and isocyanates, more preferably from the group consisting of acids, amines, azides, alcohols, and alkynes.

Advantages of this embodiment include a fast diffusion of the ligand, in particular when compared to sol-gel applications. The target is also confined in a defined/limited space, while still being in solution and able to reorient freely. It has all the advantages of immobilization on a surface while the protein remains in its native state. Furthermore, the target is freely reorienting, which allows easy implementation of NMR techniques. For instance, the NMR signal of the target can be detected which is not possible using the TINS approach.

As used herein, the term "target" or "target compound" refers to a compound, preferably a protein, enzyme, nucleic acid, polymer, antibody, antigen, part of a cell, microsome or virus, which can be modified by a ligand to change its properties. The term "ligand" or "ligand complex" refers to a compound, particularly a small organic molecule, natural compound, peptide, new chemical entity, small protein, small nucleic acid, organo-metallic compound or small polymer, which can bind to modify a target resulting in a change in the target's activity.

Other advantages of the present invention are that the required quantity of target that is necessary in a screening assay is considerably reduced. The more so, since in accordance with the present invention the target can easily be recycled, which recycling can also be automated.

In addition, the higher stability of the target allows for higher reproducibility of the screening experiments. Consequently, the number of experiments necessary to reach a statistically relevant result is reduced considerably, again reducing the quantity of target needed. Furthermore, the targets remain in their native state whether entrapped in a gel or chemically immobilized on a surface, making the assay more reliable and faster to develop and implement. Furthermore, the lifetime of the targets is increased due to protection from degrading agents such as proteases, RNases, DNases, fungi and the like. In addition, by using a capsule the macroscopic diffusion of the target is stopped while it remains free to move in its confined environment and accessible to ligands. The structural form of the target can be directly checked by NMR spectroscopy, thereby removing any uncertainty about the structure of the target.

The present invention will now be illustrated by the following

### examples.

### EXAMPLES

### Example 1: Encapsulation of the target

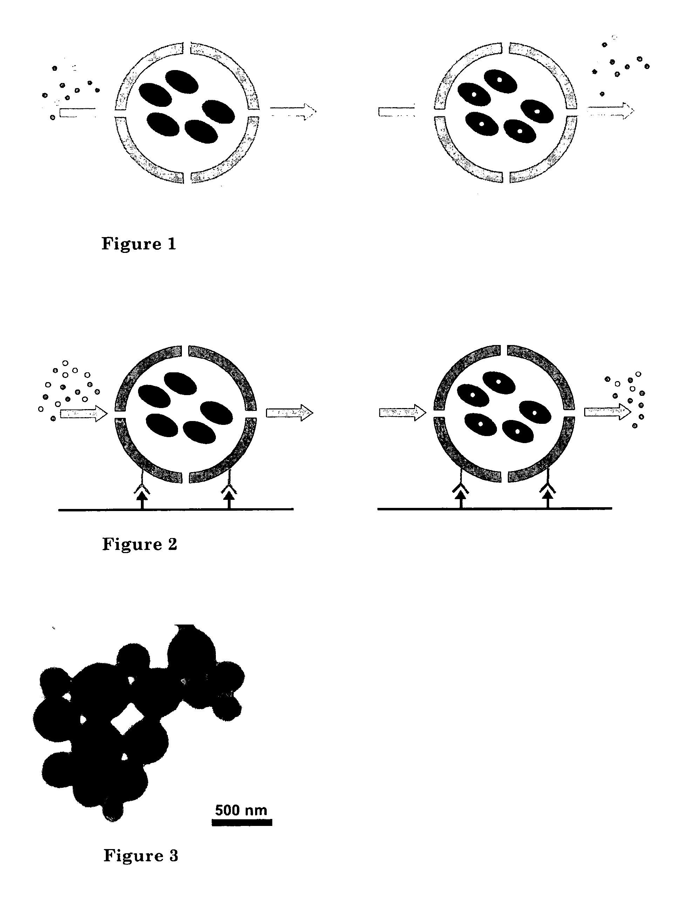

Ubiquitin was encapsulated using the block copolymer polystyrene-b-polyisocyanoalanyl thiophene, (PS-PIAT) (Vriezema et αl., Angew. Chem. Int. Ed., 2003, 42, 772-776). 200 mg of the polymer was dissolved in 100 mL THF. This was then injected into 1 mL aqueous solution of ubiquitin (500 mg/mL of PBS buffer, pH 5.2). The solution was then dialyzed overnight using a 14 kD membrane against the same PBS buffer to remove any non-encapsulated ubiquitin and the THF. The resulting capsules containing ubiquitin were then studied with electron microscopy (Figure 3) and NMR spectroscopy.

### Example 2: NMR analysis of encapsulated protein.

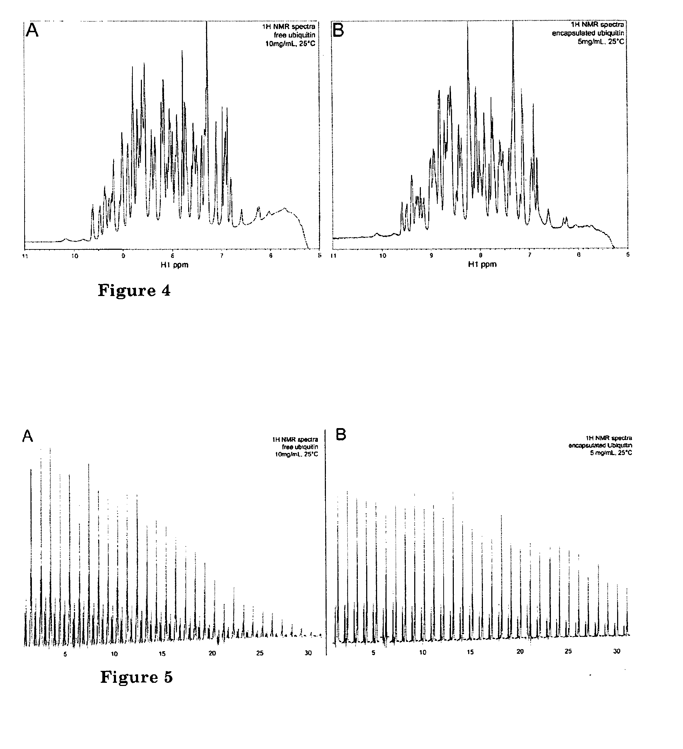

¹H NMR spectra of the free and encapsulated ubiquitin protein (prepared as described in Example 1) were recorded on a spectrometer operating at 500 MHz (proton frequency) and fitted with a cryo-cooled probe (Varian). The amide/aromatic regions of the ¹H NMR spectra are reported in Figure 4. Comparison of the ¹H NMR spectra does not reveal any significant changes in the NMR chemical shift spread and positions which justifies by itself the innocuousness of the encapsulation method in respect to the protein structure/activity.

The diffusion rates of the free and encapsulated ubiquitin were investigated using NMR gradient diffusion method. The results are reported in Figure 5 : ¹H NMR spectra are plotted against increasing NMR gradient strength (see the 31 horizontally-stacked plot spectra, Figure 5A and 5B). Using the same experimental parameters, we observed that while the NMR signal arising from the free protein has completely vanished at the higher NMR gradient strength (Figure 5A, right side of the stacked plot), a strong NMR signal is still detected for the encapsulated protein (Figure 5B, right side of the stacked plot). This unambiguously demonstrates that the encapsulated protein is indeed entrapped in the capsules which diffuse much slower than the free protein in solution. Fitting of the NMR signal intensities revealed that the encapsulated protein diffused indeed at least 4 times slower than the free protein in solution.

## Claims

1. Method for ligand screening comprising a step in which a potential ligand is brought into contact with a target compound, **characterized in that** said target is encapsulated by a semi-porous shell.

2. Method according to claim 1, followed by a detection step, which preferably comprises an analytical technique based on fluorescence spectroscopy, NMR spectroscopy, surface plasmon resonance, LC/MS-based detection method, or combinations thereof.

3. Method according to any of the previous claims, wherein the porous shell comprises at least one selected from the group consisting of an amphiphilic molecule and a polymeric material.

4. Method according to any of the previous claims, wherein the porous shell comprises at least one compound selected from the group consisting of a lipid, a homopolymer, and a block copolymer.

5. Method according to any of the previous claims, wherein the porous shell is cross-linked comprising an amphiphilic block copolymer comprising cross-linkable groups.

6. Method according to claim 5, wherein the cross-linkable groups are selected from the group consisting of thiophenes, acids, alcohols, amines, alkynes, alkenes, and azides.

7. Method according to any of the previous claims, wherein the porous shell has an average pore diameter of 0.1-5 nm, preferably 0.3-1 nm.

8. Method according to any of the previous claims, wherein the porous shell is suspended in a solvent.

9. Method according to any of the previous claims, wherein the porous shell is immobilized in a matrix, such as a sol-gel.

10. Method according to any of the previous claims, wherein the porous shell is immobilized on a surface.

11. Method according to claim 10, wherein the porous shell comprises 0.1-70 wt.%, preferably 0.5-30 wt.%, more preferably 1-15 wt.% of a functionalized block copolymer

12. Method according to claim 10, wherein the porous shell comprises 0.1-70 wt.%, preferably 0.5-30 wt.%, more preferably 1-15 wt.% of a functionalized homopolymer.

13. Method according to any one of claims 10-12, wherein the porous shell is immobilized on a surface through a functionality selected from the group consisting of acids, amines, azides, alcohols, and alkynes.

14. Method according to any of the previous claims, wherein the target is selected from the group consisting of a protein, an enzyme, a nucleic acid, a polymer, an antibody, an antigen, a part of a cell, a microsome or a virus.

15. Method according to any of the previous claims, wherein the ligand is selected from the group consisting of a small organic molecule, a natural compound, a peptide, a new chemical entity, a small protein, a small nucleic acid, an organo-metallic compound or a small polymer.
